# EUROPÄISCHE PATENTSCHRIFT

(11) **EP 0 604 792 B1**
(45) Veröffentlichungstag und Bekanntmachung des Hinweises auf die Patenterteilung: **17.04.1996**
(21) Anmeldenummer: 93119666.1
(22) Anmeldetag: 07.12.1993
(51) Int. Cl.: B01J 23/80, B01J 23/84, B01J 23/88, C07C 29/141, C07C 29/145, C07C 29/149, C07C 29/17

(54) **Kupferkatalysator**
Copper catalyst
Catalyseur en cuivre

(30) Priorität: 28.12.1992 DE 4244273
(43) Veröffentlichungstag der Anmeldung: 06.07.1994
(73) Patentinhaber: HOECHST AKTIENGESELLSCHAFT, D-65926 Frankfurt am Main (DE)
(72) Erfinder: Horn, Gerhardt, Dr. Dipl.-Chem., D-46147 Oberhausen (DE); Deckers, Gregor, Dr. Dipl.-Chem., D-46509 Xanten (DE)

(56) Entgegenhaltungen:
- EP-A- 0 125 689
- EP-A- 0 152 809
- EP-A- 0 424 069
- EP-A- 0 528 305
- US-A- 4 477 594

## Beschreibung

Die Erfindung betrifft einen neuen Katalysator, der neben Kupferoxid noch Zinkoxid und Aluminiumoxid enthält und ein verfahren zu seiner Herstellung. Der Katalysator hat sich aufgrund seiner hohen Aktivität und Selektivität bei der Hydrierung organischer Verbindungen, insbesondere bei der Hydrierung ungesättigter oder gesättigter Aldehyde, Ketone, Carbonsäuren oder Carbonsäureester zu gesättigten Alkoholen bewährt.

Kupfer enthaltende Katalysatoren finden in der chemischen Technik umfangreiche Anwendung. Entsprechend den Einsatzgebieten unterscheiden sie sich vor allem durch ihren stofflichen Aufbau und bei gleichen Bestandteilen durch die quantitative Zusammensetzung.

Speziell Kupferoxid/Zinkoxid/Aluminiumoxid-Katalysatoren sind die Grundlage der modernen Verfahren zur Synthese von Methanol aus Kohlenmonoxid und Wasserstoff bei niedrigen Drücken.

So werden in der DE-A-20 56 612 Katalysatoren für die Herstellung von Methanol beschrieben, die Verbindungen der Mischkristallreihe (CuₓZn_{y})Al₂(OH)₁₆CO₃ . 4H₂O sind, wobei x und y Zahlenwerte von 0,5 bis 5,5 annehmen können und die Summe von x und y 6 ist. Man erhält die gewünschten Mischkristalle durch Umsetzung wässriger Kupfer-, Zink- und Aluminiumnitrat enthaltender Lösungen mit basischen Reagenzien wie wässriger Natriumcarbonatlösung bei pH-Werten zwischen 4,5 und 5,5.

Die EP-A-O1 25 689 betrifft ebenfalls Kupferoxid/Zinkoxid/Aluminiumoxid-Katalysatoren für die Methanolsynthese. Sie sind durch ein Cu/Zn-Atomverhältnis von 2,8 bis 3,8 und einen Al₂O₃-Anteil von 8 bis 12 Gew.-% charakterisiert. Zu ihrer Herstellung fällt man Kupfer und Zink mit alkalisch reagierenden Substanzen wie Alkali- oder Ammoniumcarbonat in Gegenwart der Aluminiumoxidkomponente, bevorzugt kolloidal verteiltes Aluminiumoxid oder -hydroxid, gemeinsam aus ihrer wässrigen Lösung aus. Im nicht reduzierten Katalysator haben 20 bis 40 % der Poren einen Radius von 1,0 bis 3,75 nm, von 60 bis 80 % der Poren ist der Radius größer als 3,75 nm.

Ein weiteres Einsatzgebiet von Katalysatoren der genannten stofflichen Zusammensetzung sind Verfahren zur Hydrierung organischer Verbindungen. CuO/ZnO/Al₂O₃-Katalysatoren ersetzen bei diesem Reaktionstyp Kupfer/Chromoxid-Katalysatoren (bekannt als Adkins-Katalysatoren), deren Verwendung in letzter Zeit aus ökologischen Gründen vermieden wird und sie stehen im Wettbewerb mit der Vielfalt beschriebener und technisch eingeführter Nickelkatalysatoren.

Hydrierkatalysatoren auf Basis von Kupferoxid, Zinkoxid und Aluminiumoxid, deren Porenvolumen zu mindestens etwa 80 % aus Poren eines Durchmessers der größer als etwa 80 Å (8 nm) ist, sind Gegenstand der EP 04 24 069. Nach bevorzugten Ausführungsformen hat das Katalysatorpulver eine Oberfläche von wenigstens 70 m²/g, der durchschnittliche Teilchendurchmesser beträgt etwa 8 bis etwa 28 µm und das Atomverhältnis von Kupfer zu Zink ist etwa 0,2 bis etwa 5,5. Zur Herstellung der Katalysatoren bereitet man zwei wässrige Lösungen, von denen die eine Kupfer- und Zinksalz, die andere ein basisches Aluminiumsalz (z.B. Natriumaluminat) und ein basisches Fällungsreagens (z.B. Soda) enthält. Die beiden Lösungen werden gleichzeitig in einem solchen Verhältnis miteinander gemischt, daß der pH-Wert der resultierenden Mischung zumindest etwa 7 beträgt. Anschließend wird der ausgefallene Feststoff abfiltriert und kalziniert. Der Katalysator wird zur Hydrierung von Aldehyden, Ketonen, Carbonsäuren und Carbonsäureestern verwendet.

Die bekannten Kupferoxid, Zinkoxid und Aluminiumoxid enthaltenden Katalysatoren sind häufig gezielt für bestimmte Reaktionen, z.B. die Methanolsynthese oder die Hydrierung organischer Verbindungen, entwickelt worden. Sie lassen sich zwar auch für andere chemische Umsetzungen verwenden, führen aber dann nicht in allen Fällen zu optimalen Ergebnissen. Auch verschiedene Durchführungsformen derselben Reaktion können die Bereitstellung individualisierter Katalysatoren erfordern. So lehrt die Erfahrung, daß die Umsetzung derselben Reaktanten unter verschiedenen Bedingungen, z.B. in gasförmiger oder in flüssiger Form an fest angeordneten Katalysatoren oder an einem Katalysator, der im Substrat suspendiert ist, speziell angepaßte Katalysatoren voraussetzt. In diesem Zusammenhang ist zu berücksichtigen, daß bei Prozessen, die im großtechnischen Maßstab durchgeführt werden, schon die Erhöhung des Umsatzes oder die Verbesserung der Selektivität in der Größenordnung von Zehntelprozent zu bedeutsamen wirtschaftlichen Vorteilen führen kann.

Daher ist man bestrebt, Katalysatoren hoher Wirksamkeit herzustellen, die entweder möglichst umfangreich eingesetzt oder zielgerichtet für bestimmte Prozesse oder Prozeßvarianten verwendet werden können.

Die Erfindung löst die vorstehend beschriebenen Aufgaben durch einen Katalysator, der je 100 Gew.-teile Kupferoxid 40 bis 130 Gew.-teile Zinkoxid, 2 bis 50 Gew.-teile Aluminiumoxid und 1 bis 4 Gew.-teile Natriumoxid enthält und eine Gesamtoberfläche (nach BET) von 50 bis 100 m²/g besitzt, wobei 75 bis 95 % der Gesamtoberfläche von Poren mit Radien von 9 bis 1.000 nm und die restliche Gesamtoberfläche von Poren gebildet wird, deren Radius kleiner als 9 nm ist.

Der erfindungsgemäße Katalysator erweist sich Katalysatoren gleicher oder ähnlicher qualitativer Zusammensetzung hinsichtlich Aktivität und Selektivität deutlich überlegen. Er hat sich insbesondere als Hydrierkatalysator bewährt und wird mit großem Erfolg z.B. bei der Hydrierung von gesättigten oder ungesättigten Aldehyden, Carbonsäuren oder Carbonsäureestern zu gesättigten Alkoholen eingesetzt.

Bevorzugt enthält der Katalysator je 100 Gew.-teile Kupferoxid 40 bis 100 insbesondere 45 bis 80 Gew.-teile Zinkoxid, 4 bis 30, insbesondere 4 bis 20 Gew.-teile Aluminiumoxid und 1,5 bis 3 Gew.-teile Natriumoxid. Gegebenenfalls kann der Katalysator noch weitere Metalle wie Mangan, Molybdän, Vanadium, Zirkon und/oder ein Erdalkalimetall enthalten. Als Oxide, nämlich MnO, MoO₃, V₂O₅, ZrO₂ und MeO (Me steht für ein Erdalkalimetall) gerechnet, beträgt ihr Anteil je 100 Gew.-teile CuO 0,5 bis 8, bevorzugt 1 bis 6, insbesondere 2 bis 4 Gew.-teile. Die vorstehenden auf die Metalloxide bezogenen quantitativen Angaben dienen lediglich der analytischen Beschreibung des Katalysators. Sie geben seinen stofflichen Aufbau aber nicht in dem Sinne wieder, daß z.B. Natrium tatsächlich als Na₂O und Aluminium in Form der chemischen Verbindung Al₂O₃ vorliegen.

Neben der stofflichen Zusammensetzung ist für den neuen Katalysator auch die Gesamtoberfläche charakteristisch. Ihre Bestimmung erfolgt durch Adsorption von Stickstoff nach dem BET-Verfahren. Der erfindungsgemäße Katalysator hat im kalzinierten Zustand, d.h. nach 3 bis 10 stündigem Erhitzen auf 350 bis 480, vorzugsweise 400 bis 460°C eine Oberfläche von 50 bis 100, insbesondere 60 bis 80 m²/g.

Der neue Katalysator ist weiterhin durch eine bestimmte Porenstruktur gekennzeichnet. Der Anteil der Poren mit einem Radius von 9 bis 1.000 nm an der Gesamtoberfläche beträgt 75 bis 95 %, die restliche Gesamtoberfläche wird von Poren gebildet, deren Radius kleiner als 9 nm ist. Vorzugsweise liegt der Anteil der Poren mit einem Radius von 9 bis 1.000 nm bei 80 bis 90% und der Poren mit einem Radius, der kleiner als 9 nm ist bei 10 bis 20%. Besonders bewährt haben sich Katalysatoren, deren Gesamtoberfläche zu 55 bis 85 % von Poren mit einem Radius von 15 bis 1000 nm, zu 5 bis 25 % von Poren mit einem Radius von 9 bis kleiner 15 nm und zu 10 bis 20 % von Poren kleiner 9 nm gebildet wird. Auch die vorstehenden Angaben über die Porengröße beziehen sich auf den kalzinierten Katalysator.

Die Porenradienverteilung wird nach zwei Verfahren bestimmt, deren Anwendungsbereiche sich ergänzen. Die Verteilung kleiner Radien bis zu einer Größe von etwa 30 nm wird durch Auswertung der N₂-Desorptionsisothermen mit Hilfe der Kelvin-Gleichung nach C. Pierce, J.Phys. Chem. 57 (1953), 149 ff. ermittelt. Porenradien von etwa 4 nm bis zu etwa 0,1 mm bestimmt man mittels Quecksilberpenetration nach H.L. Ritter und L.C. Drake, Ind. Eng. Chem. analyt. Ed. 17 (1945), 782.

Poren der genannten Größe und Größenverteilung können durch einen hohen CO₂-Gehalt(in Form von Carbonat) in der Katalysatorvorstufe, d.h. dem bei 50 bis 95°C getrockneten, jedoch noch nicht kalzinierten Produkt erzielt werden. Es hat sich als zweckmäßig erwiesen, daß dieses Vorprodukt je 100 Gew.-teile Kupferoxid 32 bis 45, vorzugsweise 36 bis 40 Gew.-teile CO₂ enthält.

Der neue Katalysator kann grundsätzlich nach üblichen Verfahren hergestellt werden, z.B. durch Fällung der Komponenten aus wässriger Lösung unter Bedingungen, die den als zweckmäßig erkannten hohen CO₂-Anteil des Katalysatorvorproduktes sicherstellen. Ein besonders geeigneter Prozeß besteht darin, zu einer durch Umsetzung einer Aluminiumsalzlösung mit einer Alkalicarbonat- oder Alkalihydrogencarbonatlösung erhaltenen Aluminiumhydroxidsuspension bei 80 bis 100°C unter Rühren gleichzeitig aber getrennt eine Kupfer- und Zinksalz enthaltende Lösung und eine Alkalicarbonat- oder Alkalihydrogencarbonatlösung zu geben, den Feststoff abzufiltrieren, zu waschen, bei 50 bis 95°C zu trocknen und darauf bei 350 bis 480°C zu kalzinieren. Als Aluminium-, Kupfer- und Zinksalze setzt man in Wasser leicht lösliche Verbindungen ein, die sich von anorganischen oder auch organischen Säuren ableiten, wie Halogenide, Sulfate oder Acetate. Wegen ihrer großen Löslichkeit, der chemischen Indifferenz und vergleichsweise leichten Entfernbarkeit des Anions und der allgemeinen Verfügbarkeit, bevorzugt man die Nitrate. Als Fällungsmittel setzt man Alkalicarbonate und/ oder -hydrogencarbonate, insbesondere die Natriumsalze ein. Die Konzentration der Salze in den Lösungen kann sich über weite Bereiche erstrecken. Die Aluminiumsalzlösung enthält je Liter Lösung das Aluminiumsalz in einer Menge, die 20 bis 100, vorzugsweise 30 bis 90 und insbesondere 40 bis 80 g Al₂O₃ entsprechen.

Die Konzentrationen von Kupfer und Zink in ihrer gemeinsamen Lösung betragen 10 bis 100 g Cu und 5 bis 80 g Zn je Liter Lösung. Das als Fällungsmittel bevorzugte Natriumcarbonat wird zweckmäßig in Form einer Lösung, die 50 bis 150 g Na₂CO₃ je Liter enthält, eingesetzt, wenngleich auch niedrigere Natriumcarbonatkonzentrationen nicht ausgeschlossen sind.

Zur Ausfällung des Aluminiumhydroxids läßt man innerhalb von 2 bis 10 min eine auf 20 bis 90°C temperierte Aluminiumsalzlösung in eine Alkalicarbonat- und/oder Alkalihydrogencarbonatlösung, deren Temperatur 20 bis 100°C, vorzugsweise 30 bis 80°C beträgt, unter intensivem Rühren einfließen. Das Mengenverhältnis von Aluminiumsalz und Fällungsmittel wird so gewählt, daß nach beendeter Umsetzung der pH-Wert der Suspension 6,5 bis 8,5 ist. Anschließend stellt man die Temperatur der Suspension auf 80 bis 100°C, vorzugsweise 90 bis 98°C ein und gibt zu ihr innerhalb von 10 bis 30 min unter kräftigem Rühren, gleichzeitig aber getrennt, die jeweils auf 80 bis 100°C, vorzugweise 90 bis 98°C erhitzte Alkalicarbonat- und/oder Alkalihydrogencarbonatlösung und die Kupfersalz-/Zinksalzlösung. Die Zuflußmengen der beiden Lösungen werden so aufeinander abgestimmt, daß der pH-Wert der Fällungssuspension im Bereich von 7,5 bis 8,0 konstant bleibt. Sobald die Zugabe der beiden Lösungen beendet ist, filtriert man die Suspension. Der Filterrückstand wird gewaschen, bei 50 bis 95°C getrocknet und anschließend bei 350 bis 480°C, vorzugsweise 400 bis 460°C kalziniert. Die Formung der Masse, z.B. durch Extrusion oder Tablettieren, kann vor der Trocknung oder nach der Kalzinierung erfolgen. Der Katalysator wird durch Reduktion bei 130 bis 200°C, vorzugsweise 150 bis 180°C aktiviert. Die Reduktion nimmt man in einem separaten Reaktor oder unmittelbar im Hydrierreaktor vor.

Der neue Katalysator wird mit Erfolg zur Hydrierung organischer Verbindungen verwendet, vorzugsweise zur Hydrierung ungesättigter oder gesättigter Aldehyde, Ketone, Carbonsäuren oder Carbonsäureester. Besonders bewährt hat er sich bei der Hydrierung ungesättigter und gesättigter Aldehyde in der Gasphase.

Die folgenden Beispiele erläutern die Erfindung ohne sie zu beschränken.

### Beispiel 1: Katalysator-Herstellung

Zu einer auf 70°C erhitzten Lösung von 580 g Soda in 5,5 l Wasser läßt man unter kräftigem Rühren innerhalb von 3 Minuten eine auf Raumtemperatur gehaltene Lösung von 925 g Al(NO₃)₃·9 H₂O in 3 l Wasser einfließen. Unter fortgesetztem Rühren erhöht man innerhalb von etwa 10 min die Temperatur der erhaltenen Suspension auf 95°C und versetzt sie innerhalb von 15 min gleichzeitig aber getrennt mit einer auf 95°C erhitzten Lösung von 2249,2 g Cu(NO₃)₂·3 H₂O und 1755,4 g Zn(NO₃)₂·6 H₂O in 10 l Wasser und einer ebenfalls auf 95°C erhitzten Lösung von 2340 g Na₂CO₃ in 18 l Wasser. Während der Umsetzung hält man in der Fällsuspension einen pH-Wert von 7,5 bis 8,0 aufrecht. Anschließend wird die Suspension weitere 2 min gerührt und darauf filtriert. Der Filterrückstand wird in 80 min kontinuierlich mit 80 l Wasser gewaschen und nach Verformung durch Extrusion oder durch Sprühtrocknung bei maximal 100°C bis auf einen Gehalt von weniger als 5 % Wasser getrocknet.

Das getrocknete, unkalzinierte Katalysatorvorprodukt enthält 38,8 Gew.-% CuO (31 Gew.-% Cu) und je 100 Gew.-teile CuO, 65 Gew.-teile ZnO, 17 Gew.-teile Al₂O₃, 2,0 Gew.-teile Na₂O und 38,6 Gew.-teile CO₂.

In einem anschließenden Verfahrensschritt wird das Vorprodukt im Stickstoff- oder Luftstrom (200 l/h) auf 430°C erhitzt und weitere 3 h bei dieser Temperatur gehalten. Es resultiert ein kalziniertes Produkt, das 51 Gew.-% CuO und je 100 Gew.-teile CuO, 65 Gew.-teile ZnO, 17 Gew.-teile Al₂O₃, 2,0 Gew.-teile Na₂O und 1,5 -Gew.-teile CO₂ enthält. Es hat eine Gesamtoberfläche (nach BET) von 68 m²/g; 84 % der Oberfläche werden von Poren mit Radien die größer als 9 nm und 16 % von Poren mit Radien die kleiner als 9 nm sind, gebildet.

Das kalzinierte Produkt kann extrudiert oder tablettiert in Form eines Festbettes als Katalysator eingesetzt werden.

### Beispiel 2: Hydrierung von n-Butyraldehyd

250 ml des nach Beispiel 1 hergestellten und tablettierten Katalysators werden in einem Rohrreaktor bei 160°C mit 400 l/h eines H₂-/N₂-Gemisches, das 3 Vol.-% H₂ enthält, solange reduziert, bis kein Wasser mehr gebildet wird. Anschließend leitet man bei 145°C und einem Überdruck von 0,3 MPa ein Gemisch aus 250 ml dampfförmigem n-Butyraldehyd und 710 l Wasserstoff je Stunde über den Katalysator. Der überschüssige Wasserstoff wird im Kreislauf geführt.

Das Hydrierprodukt ist nach gaschromatographischer Analyse wie folgt zusammengesetzt:

| | |
|---|---|
| n-Butanol | 99,8 Gew.-% |
| n-Butyraldehyd | < 0,05 Gew.-% |
| Buttersäure-butylester | < 0,05 Gew.-% |
| 2-Ethylhexanol | < 0,05 Gew.-% |
| Di-n-butylether | ≦ 10,0 Gew.-ppm |

Besonders bemerkenswert ist die im Vergleich zum Stand der Technik äußerst geringe Bildung von Nebenprodukten bei Einsatz der erfindungsgemäßen Katalysatoren.

### Beispiel 3: Hydrierung von 2-Ethylhexenal

3 l des nach Beispiel 1 hergestellten und tablettierten Katalysators werden wie im Beispiel 2 beschrieben reduziert. Anschließend leitet man über ihn bei 145°C und einem Überdruck von 50 kPa ein dampfförmiges Gemisch aus 1500 ml/h 2-Ethylhexenal (flüssig) und 4,4 Nm³/h Wasserstoff. Einsatzmaterial und Reaktionsprodukt sind nach gaschromatographischer Analyse wie folgt zusammengesetzt:

| | Einsatzmaterial (Gew.-%) | Reaktionsprodukt (Gew.-%) |
|---|---|---|
| C₇-/C₈-Kohlenwasserstoffe | ≦ 0,1 | ≦ 0,1 |
| n-/i-Butanal | 3 - 4 | - |
| n-/i-Butanol | 0,5 - 1,5 | 3,5 - 4,5 |
| 2-Ethylhexenal | 90 - 91,5 | ≦ 0,1 |
| 2-Ethylhexanal | etwa 1 | 0,2 - 0,3 |
| 2-Ethylhexanol | 3,5 - 4,5 | 95 - 97 |
| höher siedende Anteile (wie Acetale, Diole) | 2 - 3 | 0,5 - 0,8 |

### Beispiel 4 (Vergleich): Hydrierung von n-Butyraldehyd

In diesem Beispiel wird ein Katalysator des Standes der Technik eingesetzt. Er enthält 47,5 Gew.-% Cu und je 100 Gew.-teile CuO, 49,5 Gew.-teile ZnO, 8,4 Gew.-teile Al₂O₃ und 0,12 Gew.-teile Na₂O. Seine Gesamtoberfläche (nach BET) beträgt 128 g/m²; 85 % der Oberfläche werden von Poren mit einem Radius der kleiner oder gleich 15 nm ist, gebildet.

250 ml des tablettierten Katalysators werden in einem Rohrreaktor, wie im Beispiel 2 beschrieben, reduziert. Anschließend leitet man bei 145°C und einem Überdruck von 0,3 MPa ein dampfförmiges Gemisch aus 250 ml/h n-Butyraldehyd (flüssig) und 710 l Wasserstoff je Stunde über den Katalysator. Der überschüssige Wasserstoff wird im Kreislauf geführt.

Das Hydrierprodukt ist nach gaschromatographischer Analyse wie folgt zusammengesetzt:

| | |
|---|---|
| n-Butanol | 99,5 - 99,6 Gew.-% |
| n-Butyraldehyd | 0,2 - 0,3 Gew.-% |
| Buttersäure-butylester | etwa 0,02 Gew.-% |
| Di-n-butylether | etwa 100 Gew.-ppm |

## Patentansprüche

1. Katalysator, der je 100 Gew.-teile Kupferoxid 40 bis 130 Gew.-teile Zinkoxid, 2 bis 50 Gew.-teile Aluminiumoxid und 1 bis 4 Gew.-teile Natriumoxid enthält und eine Gesamtoberfläche von 50 bis 100 m²/g, nach BET, besitzt, wobei 75 bis 95 % der Gesamtoberfläche von Poren mit Radien von 9 bis 1.000 nm und die restliche Gesamtoberfläche von Poren, deren Radius kleiner als 9 nm ist, gebildet wird.

2. Katalysator nach Anspruch 1, dadurch gekennzeichnet, daß er je 100 Gew.-teile Kupferoxid 40 bis 100, insbesondere 45 bis 80 Gew.-teile Zinkoxid, 4 bis 30, insbesondere 4 bis 20 Gew.-teile Aluminiumoxid und 1,5 bis 3 Gew.-teile Natriumoxid enhält.

3. Katalysator nach den Ansprüchen 1 oder 2, dadurch gekennzeichnet, daß er je 100 Gew.-Teile Kupferoxid 0,5 bis 8 Gew.-teile Mangan, Molybdän, Vanadium, Zirkon und/oder ein Erdalkalimetall, gerechnet als MnO, MoO₃, V₂O₅, ZrO₂, MeO, Me = Erdalkalimetall, enthält.

4. Katalysator nach Anspruch 3, dadurch gekennzeichnet, daß er 1 bis 6, insbesondere 2 bis 4 Gew.-teile der Metalle Mangan, Molybdän, Vanadium, Zirkon und/oder Erdalkalimetall, gerechnet als MnO, MoO₃, V₂O₅, ZrO₂, MeO, Me = Erdalkalimetall, enthält.

5. Katalysator nach einem oder mehreren der Ansprüche 1 bis 4, dadurch gekennzeichnet, daß er eine Oberfläche von 60 bis 80 m²/g, nach BET, besitzt.

6. Katalysator nach einem oder mehreren der Ansprüche 1 bis 5, dadurch gekennzeichnet, daß 80 bis 90 % der Gesamtoberfläche von Poren mit einem Radius von 9 bis 1000 nm und 10 bis 20 % der Gesamtoberfläche von Poren mit einem Radius, der kleiner 9 nm ist, gebildet werden.

7. Katalysator nach einem oder mehreren der Ansprüche 1 bis 6, dadurch gekennzeichnet, daß 55 bis 85 % der Gesamtoberfläche von Poren mit einem Radius von 15 bis 1000 nm, 5 bis 25 % der Gesamtoberfläche von Poren mit einem Radius von 9 bis kleiner 15 nm und 10 bis 20 % der Gesamtoberfläche von Poren mit einem Radius kleiner 9 nm gebildet werden.

8. Verfahren zur Herstellung eines Katalysators nach einem oder mehreren der Ansprüche 1 bis 7, dadurch gekennzeichnet, daß man zu einer durch Umsetzung einer Aluminiumsalzlösung mit einer Alkalicarbonat- und/oder Alkalihydrogencarbonatlösung erhaltenen und auf 80 bis 100°C erhitzten Aluminiumhydroxidsuspension unter Rühren gleichzeitig aber getrennt eine auf 80 bis 100°C erhitzte Kupfer- und Zinksalz enthaltende Lösung und eine auf 80 bis 100°C erhitzte Alkalicarbonat- oder Alkalihydrogencarbonatlösung unter Aufrechterhaltung eines pH-Wertes von 7,5 bis 8,0 in der Fällungssuspension gibt, den Feststoff abfiltriert, wäscht, bei 50 bis 95°C trocknet und darauf bei 350 bis 480°C kalziniert.

9. Verfahren nach Anspruch 8, dadurch gekennzeichnet, daß zur Herstellung der Aluminiumhydroxidsuspension eine Aluminiumsalzlösung mit einer Temperatur von 20 bis 90°C unter intensivem Rühren zu einer Alkalicarbonat- und/oder Hydrogencarbonatlösung mit einer Temperatur von 20 bis 100°C in einer solchen Menge gegeben wird, daß nach beendeter Umsetzung der pH-Wert der Suspension 6,5 bis 8,5 beträgt.

10. Verwendung des Katalysators nach einem oder mehreren der Ansprüche 1 bis 7 zur Hydrierung organischer Verbindungen, insbesondere zur Hydrierung ungesättigter oder gesättigter Aldehyde, Ketone, Carbonsäuren oder Carbonsäureester zu gesättigten Alkoholen.

## Claims

1. A catalyst comprising per 100 parts by weight of copper oxide from 40 to 130 parts by weight of zinc oxide, from 2 to 50 parts by weight of aluminum oxide and from 1 to 4 parts by weight of sodium oxide and having a total surface area by BET of from 50 to 100 m²/g, in which from 75 to 95% of the total surface area is made up by pores having radii of from 9 to 1000 nm and the remainder of the total surface area is made up by pores having a radius of less than 9 nm.

2. A catalyst as claimed in claim 1, comprising per 100 parts by weight of copper oxide from 40 to 100, in particular from 45 to 80, parts by weight of zinc oxide, from 4 to 30, in particular from 4 to 20, parts by weight of aluminum oxide and from 1.5 to 3 parts by weight of sodium oxide.

3. A catalyst as claimed in claim 1 or 2, comprising per 100 parts by weight of copper oxide from 0.5 to 8 parts by weight of manganese, molybdenum, vanadium, zirconium and/or an alkaline earth metal, calculated as MnO, MoO₃, V₂O₅, ZrO₂, MeO, Me = alkaline earth metal.

4. A catalyst as claimed in claim 3, comprising from 1 to 6, in particular from 2 to 4, parts by weight of the metals manganese, molybdenum, vanadium, zirconium and/or an alkaline earth metal, calculated as MnO, MoO₃, V₂O₅, ZrO₂, MeO, Me = alkaline earth metal.

5. A catalyst as claimed in one or more of claims 1 to 4, having a surface area of from 60 to 80 m²/g by BET .

6. A catalyst as claimed in one or more of claims 1 to 5, wherein from 80 to 90% of the total surface area is made up by pores having a radius of from 9 to 1000 nm and from 10 to 20% of the total surface area is made up by pores having a radius less than 9 nm.

7. A catalyst as claimed in one or more of claims 1 to 6, wherein from 55 to 85% of the total surface area is made up by pores having a radius of from 15 to 1000 nm, from 5 to 25% of the total surface area is made up by pores having a radius of from 9 to less than 15 nm and from 10 to 20% of the total surface area is made up by pores having a radius less than 9 nm.

8. A process for preparing a catalyst as claimed in one or more of claims 1 to 7, which comprises forming an aluminum hydroxide suspension by reaction of an aluminum salt solution with an alkali metal carbonate or alkali metal hydrogen carbonate solution, simultaneously but separately adding while stirring a solution heated to from 80 to 100°C containing copper and zinc salts and a solution heated to from 80 to 100°C of alkali metal carbonate or alkali metal hydrogen carbonate while maintaining a pH of 7.5 to 8.0 in the precipitation suspension, filtering off the solid, washing, drying at from 50 to 95°C and then calcining at from 350 to 480°C.

9. The process as claimed in claim 8, wherein the aluminum hydroxide suspension is prepared by adding an aluminum salt solution having a temperature of from 20 to 90°C while stirring vigorously to an alkali metal carbonate and/or hydrogen carbonate solution having a temperature of from 20 to 100°C in such an amount that after completion of the reaction the pH of the suspension is from 6.5 to 8.5.

10. A method of using the catalyst as claimed in one or more of claims 1 to 7 for hydrogenating organic compounds, in particular for hydrogenating unsaturated or saturated aldehydes, ketones, carboxylic acids or carboxylic esters to form saturated alcohols.

## Revendications

1. Catalyseur contenant, pour 100 parties en poids d'oxyde de cuivre, 40 à 130 parties en poids d'oxyde de zinc, 2 à 50 parties en poids d'alumine et 1 à 4 parties en poids d'oxyde de sodium et ayant une surface totale de 50 à 100 m²/g selon BET, 75 à 95 % de la surface totale étant constitués de pores de rayons 9 à 1 000 nm et le reste de la surface totale de pores dont le rayon est inférieur à 9 nm.

2. Catalyseur selon la revendication 1, caractérisé en ce qu'il contient, pour 100 parties en poids d'oxyde de cuivre, 40 à 100, plus spécialement 45 à 80 parties en poids d'oxyde de zinc, 4 à 30, plus spécialement 4 à 20 parties en poids d'alumine et 1,5 à 3 parties en poids d'oxyde de sodium.

3. Catalyseur selon la revendication 1 ou 2, caractérisé en ce qu'il contient, pour 100 parties en poids d'oxyde de cuivre, 0,5 à 8 parties en poids de manganèse, de molybdène, de vanadium, de zirconium et/ou d'un métal alcalino-terreux, exprimé en MnO, MoO₃, V₂O₅, ZrO₂, MeO, Me représentant un métal alcalino-terreux.

4. Catalyseur selon la revendication 3, caractérisé en ce qu'il contient de 1 à 6, plus spécialement de 2 à 4 parties en poids des métaux manganèse, molybdène, vanadium, zirconium et/ou d'un métal alcalino-terreux, exprimé en MnO, MoO₃, V₂O₅, ZrO₂, MeO, Me représentant un métal alcalino-terreux.

5. Catalyseur selon une ou plusieurs des revendications 1 à 4, caractérisé en ce qu'il a une surface de 60 à 80 m²/g selon BET.

6. Catalyseur selon une ou plusieurs des revendications 1 à 5, caractérisé en ce que 80 à 90 % de la surface totale sont constitués de pores de rayons 9 à 1 000 nm et 10 à 20 % de la surface totale de pores de rayons inférieurs à 9 nm.

7. Catalyseur selon une ou plusieurs des revendications 1 à 6, caractérisé en ce que 55 à 85 % de la surface totale sont constitués de pores de rayons 15 à 1 000 nm, 5 à 25 % de la surface totale de pores de rayons 9 à moins de 15 nm et 10 à 20 % de la surface totale de pores de rayons inférieurs à 9 nm.

8. Procédé de préparation d'un catalyseur selon une ou plusieurs des revendications 1 à 7, caractérisé en ce que, partant d'une suspension d'hydroxyde d'aluminium obtenue par réaction d'une solution d'un sel d'aluminium avec une solution d'un carbonate et/ou d'un bicarbonate alcalin et chauffée à des températures de 80 à 100°C, on ajoute sous agitation, simultanément mais séparément, une solution contenant un sel de cuivre et un sel de zinc, chauffée à des températures de 80 à 100°C, et une solution d'un carbonate ou bicarbonate alcalin, également chauffée à des températures de 80 à 100°C, en maintenant un pH de 7,5 à 8,0 dans la suspension de précipitation puis on filtre la substance solide, on la lave, on la sèche à des températures de 50 à 95°C et on la calcine à des températures de 350 à 480°C.

9. Procédé selon la revendication 8, caractérisé en ce que, pour la préparation de la suspension d'hydroxyde d'aluminium, on ajoute sous agitation énergique une solution de sel d'aluminium à une température de 20 à 90°C à une solution de carbonate et/ou de bicarbonate alcalin à une température de 20 à 100°C, en quantité telle qu'après la réaction le pH de la suspension soit de 6,5 à 8,5.

10. Utilisation du catalyseur selon une ou plusieurs des revendications 1 à 7 pour l'hydrogénation de composés organiques, en particulier pour l'hydrogénation d'aldéhydes, cétones, acides carboxyliques ou esters d'acides carboxyliques saturés ou insaturés en alcools saturés.
